(19) Europäisches Patentamt
European Patent Office
Office européen des brevets

(11) EP 2 956 178 B1

(12) **EUROPEAN PATENT SPECIFICATION**

(45) Date of publication and mention
of the grant of the patent:
**04.04.2018 Bulletin 2018/14**

(51) Int Cl.:
*A61L 15/60* (2006.01)     *A61F 13/02* (2006.01)
*C08B 15/00* (2006.01)

(21) Application number: **14710982.1**

(22) Date of filing: **12.02.2014**

(86) International application number:
**PCT/IB2014/058934**

(87) International publication number:
**WO 2014/125418 (21.08.2014 Gazette 2014/34)**

(54) **BIODEGRADABLE SUPERABSORBENT HYDROGELS**

BIOLOGISCH ABBAUBARE SUPERABSORBIERENDE HYDROGELE

HYDROGELS SUPERABSORBANTS BIODÉGRADABLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.02.2013 IT RM20130088**

(43) Date of publication of application:
**23.12.2015 Bulletin 2015/52**

(73) Proprietor: **Jaber Innovation S.r.l.**
**00144 Rome (IT)**

(72) Inventors:
• **COMETA, Stefania**
**00144 Rome (IT)**

• **MILESI, Domenico**
**00136 Rome (IT)**
• **IANNACCONE, Giuseppe**
**00144 Rome (IT)**

(74) Representative: **Di Giovine, Paolo et al**
**Società Italiana Brevetti S.p.A.**
**Piazza di Pietra, 38-39**
**00186 Roma (IT)**

(56) References cited:
**US-A- 5 352 480      US-A1- 2004 025 798
US-A1- 2005 038 199      US-A1- 2008 082 068**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention is directed to natural polymer-based biodegradable superabsorbent hydrogels and to methods for making them. These hydrogels can be employed in hygienic-health products, in the sector of food product packaging and in medical products.

STATE OF THE PRIOR ART

Polymer hydrogels are composite materials which absorb a high amount of liquid with regard to their mass, consisting of a lattice of solid polymer and an interstitial aqueous phase. In particular, those hydrogels able to absorb a water amount greater than 95% of their overall weight are referred to as "superabsorbent". Hydrogels are often defined as two-component systems, as consisting of crosslinked polymeric chains and water; these systems are capable of absorbing and retain thereinside high amounts of aqueous solution due to the presence, on the polymer chains, of basic or acidic groups fostering hydration of the internal regions.

The process of crosslinking the polymer in solution, which leads to formation of permanent water-swollen compartments (pores) and therefore to formation of the actual hydrogel, is not spontaneous but can be triggered by external agents. There are two main crosslinking methods: physical crosslinking and chemical crosslinking. Physical crosslinking consists in adjusting parameters such as temperature, pressure, volume, ionic strength of the solution so as to trigger gelation processes via the creation, for instance, of ionic and hydrophobic interactions; these procedures (some of which difficult to carry out) are very convenient, as they enable crosslinking without addition of chemical agents (linkers), guaranteeing the integrity of substances that are incorporated inside the hydrogel.

One of the drawbacks presented by physical hydrogels, in the literature oft-times referred to as reversible hydrogels, is the presence thereinside of a high degree of dishomogeneity due to the formation, among polymer chains, of domains created following hydrophobic or ionic interactions. Physical methods most used are crosslinking by ion addition, crystallization and crosslinking by hydrogen bonds. Chemical crosslinking of a hydrogel consists in the creation of covalent bonds among polymer chains; techniques most used, apart from reactions induced by chemical reagents, are gamma irradiation and UV irradiation, which guarantee short crosslinking times and prove particularly convenient as avoiding the use of further reagents and conferring to the hydrogel optimal mechanical properties for numerous applications. The substantial difference between chemical hydrogels and physical ones (the common gels) resides in their behavior in the presence of aqueous solutions: in case of common gels, given the absence of strong bonds among the chains, a complete in-water solubilization is had, whereas chemical hydrogels are insoluble due to the existing covalent bonds.

In case of chemical hydrogels, their behavior in aqueous solution proves very interesting since the crosslinked structure lends itself to incorporate/release high amounts of solution via diffusive processes (hydrogel swelling); the amount of diffused solution will depend on the number and average size of the pores and on how such compartments are interconnected.

Such features are hardly quantifiable with precision; the methods most used to have an estimate of the geometric features of the pores consist in following the diffusion processes of probes with well-defined molecular weight or size inside the matrix. In fact, the diffusion features of such processes (characteristic times, amount of molecules incorporated, etc.), combined with the hydrophilic or hydrophobic features of the probes, can provide valuable information on the internal environment of the hydrogel.

Highly absorbent polymers, commonly defined as *"superabsorbent polymers"* (SAPs), are hydrophilic three-dimensional structures capable of absorbing water amounts of 10 up to 1000 times their dry weight. They are widely employed in various fields, like the hygienic-health, food, biomedical, cosmetic ones and in agriculture. One fundamental feature that such SAPs must possess, besides the high absorbing properties, is the absence of products or by-products that be toxic, or in some way irritating when in contact with biological systems, where SAPs employment be in the field of personal hygiene products or in the food or pharmaceutical sector.

In spite of these requirements being for the most part met by common polyacrilic-based SAPs, today the ever more pressing problem of using biodegradable materials, having however adequate efficiency in absorbing liquids, poses itself. Consumer-expressed preference for ecological products, at least in the disposable products sector, still remains an unsatisfied demand. In this scenario, the research for SAPs alternative to commercial ones, that be capable of conjugating high performances, in terms of fluid absorption and retention, with total safety of raw materials employed and the possibility of being easily disposed of or recycled, without accumulating in the environment, represents a much sought-after goal both in the academic and industrial research sector.

The use of sodium carboxymethylcellulose (CMCNa) along with hydroxyethylcellulose (HEC) for the synthesis of biodegradable SAPs was first proposed in 1990 by Anbergen and Oppermann [1], who used dimethyl sulfone as a crosslinking agent (crosslinker).

Subsequently, other crosslinking agents such as epichlorhydrin, diepoxides, carbodiimmides, etc., were employed [2]. However, all of the abovementioned crosslinking agents entail the drawback of being potentially or declaredly toxic, or of producing toxic reaction by-products. Patent Application US 2008/082068 discloses an absorbent article, as a diaper, comprising a superabsorbent material. The superabsorbent material is obtained by crosslinking a 4.5 % w/w aqueous solution of CMC in the presence of aluminium acetate in a concentration of 1.5 % of CMC. The pH of the solution is adjusted to 7.7 using NaOH. The crosslinking is performed at room temperature for 50 minutes and then the slurry is filtered and then oven dried at 76 °C for 50 minutes. The aluminium acetate is stabilized by an undisclosed amount of boric acid. PCT Patent Application WO2012170682 [3] describes the use of polycarboxylic acids, like, e.g., citric acid, to obtain a crosslinking of cellulose derivatives chains (in particular sodium carboxylmethylcellulose, CMCNa, and hydroxyethylcellulose, HEC), developing a highly absorbing hydrogel, whose performances are improved following a drying process by phase inversion in acetone. Said material, though remarkably attractive from the standpoint of raw materials employed and product performances, entails criticality aspects from the applicative standpoint, above all when one considers its large-scale production. In particular, the following drawbacks can be identified:

1) the use of crosslinking agents such as citric acid or the like, such as bi- tri- or tetra carboxylic organic acids, does not enable a precise determination of the degree of crosslinking, nor a qualitative-type investigation through conventional analytic techniques (of FT-IR, XPS, SEM-EDX type, etc.), given the similarity of the functional groups present in the crosslinking agent with those present in the substrate to be crosslinked. Therefore, a precise characterization of the obtained product is not possible.

2) the production of such materials occurs by dissolution of the cellulosic substrate in water, in diluted concentrations. From such solutions, the solvent must then be necessarily removed by a pre-evaporation process, under bland conditions (at room temperature). This reaction stage, needed so that the next stage (i.e., the actual crosslinking) may take place, where it is strictly necessary to eliminate water so that esterification may take place, requires rather long times and remarkable amounts of power in order to evaporate all the water;

3) the phase-inversion process in low-boiling organic solvents, to be able to perform the drying of the previously water-swollen product, requires remarkable amounts of solvents, with environmental issues, as well as issues of effectiveness of the process on a large scale.

One aim of the present invention is to provide superabsorbent hydrogels that be hypoallergenic, biodegradable, deriving from renewable sources and that solve the above-mentioned drawbacks.

## SUMMARY OF THE INVENTION

The solution proposed in the following invention is a method for making superabsorbent polymers comprising the following steps:a) preparing an aqueous solution at pH equal or greater than 7.5 comprising alkylcellulose and at least one crosslinking agent, wherein said alkylcellulose has a concentration by weight between 2 and 10% and wherein said crosslinking agent is selected from boric acid, boronic acid, borax or an ester thereof in a concentration between 2 and 8% of said alkylcellulose;b) performing the crosslinking reaction obtaining a gel;c) drying the gel. The method of the present invention and the superabsorbent polymers obtainable with such method entail numerous advantages, among which:

- Selection of boric acid or derivatives thereof does not produce environmental pollutants, nor substances toxic to humans; moreover, boric acid has been proven to have beneficial effects on plant growth;
- Easy assessment of occurred crosslinking reaction is possible, as in the chemical investigation of the material Boron represents a marker element detectable by various techniques;
- The pre-evaporation stage for removing water, though being a stage anyhow affording advantages in terms of performance of the obtained gel, is not strictly necessary to obtain high-efficiency gels, and the crosslinking reaction starts within reasonably shorter times;
- No improvement in hydrogel performance is observed following phase inversion, indicating that said process is not essential to obtain superabsorbent particles with superior performance, as instead indicated in the state of the art. This aspect represents an important advantage, above all for industrial-scale applications. Moreover, Boron presence in said materials was highlighted by XPS analyses conducted on particles obtained according to the method of the present invention; said presence is not found anymore in the same particles following a phase-inversion process, which therefore proves to be a drying method injurious to boric acid-based hydrogels.
- The concomitant use of boric acid and other inorganic reagents that be capable of increasing the pH of the aqueous solution in order to obtain borate ions, those really responsible for the crosslinking process, entails particular advantages if as alkalinizing agent a system like sodium hydrogen carbonate is preferred, which concomitantly also acts as porogenic agent able to create porosities in the gel mass obtained.

Therefore, a first object of the present invention is a method for making superabsorbent polymers as defined in claim 1.

A second object of the invention are the superabsorbent polymers obtainable according to the method of claim 1. A third object of the invention is an absorbent product for absorbing body fluids, characterized by the fact that its absorbent core comprises the polymers obtainable according to the method of claim 1. A fourth object of the invention is a pharmaceutical composition comprising one or more active principles and polymers obtainable according to the method of claim 1. A fifth object of the invention are absorbent products for food packaging comprising polymers obtainable according to the method of claim 1.

BRIEF DESCRIPTION OF THE FIGURES

Figure 1: DSC analysis of starting CMC, for selection of the reaction temperature (the convention used is: endothermal processes = upward peaks).
Figure 2: FT-IR Spectrum in ATR mode of the hydrogel in the absence (a) and in the presence (b) of spacers, according to the embodiment of examples 2 and 3.
Figure 3: Optical microscope image of the hydrogels of example 2 and example 5.

DETAILED DESCRIPTION OF THE INVENTION

The present invention is composed of the following aspects, described in detail below. The method comprises a first step a) in which it is prepared an alkaline aqueous solution at pH greater than 7.5, preferably between 7.5 and 9, comprising an alkylcellulose as defined in claim 1 and at least one crosslinking agent selected from boric acid, boronic acid, borax and/or esters thereof, and a step b) in which the solution, properly homogenized, can be pre-dried beforehand or directly brought to a temperature suitable for crosslinking, in preset times, to the obtainment of a compact gel.

Said alkylcellulose is selected from methylcellulose, ethylcellulose, hydroxyethylcellulose, methylhydroxyethylcellulose, methylhydroxypropylcellulose, carboxymethylcellulose or mixtures thereof, and prepared so as to obtain a concentration by weight between 2 and 20% by weight of said aqueous solution, preferably between 2 and 5% thereof. Examples of suitable carboxyalkil celluloses have a degree of substitution of the carboxyl groups between 0.4 and 2.5 and comprise 60-99% by weight of carboxyalkyl cellulose, based on the total weight of the cellulose.

The solution will also comprise boric acid, boronic acid, borax and/or esters thereof in a concentration between 2 and 15% of said alkylcellulose, preferably between 4 and 6% thereof.

Temperature during the crosslinking is not particularly critical, but generally it could range between room temperature and 80°C, preferably between 50 and 80°C, whereas crosslinking times can range between 1 and 24 hours, preferably between 3 and 7 hours.

In one embodiment, the solution could further comprise a spacer molecule selected from mono- and/or disaccharides (e.g., glucose, levulose, sucrose, fructose, glycerol, mannitol, lactose, xylitol, sorbitol, arabitol, erythritol, isomalt and cellobiose) and/or polyols (such as, e.g., polyethers and polyesters, polyvinyl alcohol, polyethylene glycol diol, polycaprolactone diol) to form a mesh having a high ability to absorb and retain water or aqueous fluids therein.

According to one embodiment, the method provides the employ of suitable organic and/or inorganic fillers capable of absorbing water. Absorbent fillers can be advantageously incorporated in the absorbent material to increase the degree of hydration thereof and as a reinforcement, especially to obtain improvements in the response of the gel subjected to external pressure. Such fillers can include microcrystalline cellulose, talc, kaolin, silica and silicates and/or clays, preferably said fillers will be bentonites and/or laponites.

Bentonites are a hydrosilicate of aluminum and magnesium. Morphologically, they are made up of alumina octahedra between two layers of tetrahedrical silica. The elementary particle has the characteristic "lamella" shape and each lamella has a distribution of negative charges on the external surface. The lamellas are held together in packets thanks to the action of electrostatic forces with cations of various type. In one embodiment, there will advantageously be used sodium bentonite, which is an activated bentonite, whose properties are achieved by chemical processes in which the presence of Sodium cation ($Na^+$) is artificially increased compared to the other cations. Laponites™ are instead synthetic silicates that in water, in the presence of other solids or electrolytes, are able to form gel. Compared to bentonite (which is a natural, non-synthetic silicate) markedly lower sizes are had. In this invention, laponites with $\leq$250 nm particle sizes were employed.

The alkaline pH of the solution could be obtained with one or more alkalinizing agents, like, e.g., NaOH. In one preferred embodiment, $NaHCO_3$ will be used as alkalinizing agent. It was surprisingly observed that, by utilizing $NaHCO_3$, it is possible to obtain polymers with a higher absorption efficiency.

While not wishing to bind the present invention to any mechanism, it may be surmised that the improved efficiency obtained in the presence of $NaHCO_3$ be due to the fact that, besides acting as alkalinizing agent necessary in the stage of transformation of boric acid into borate, which is the active crosslinking species in said crosslinking reaction, sodium hydrogen carbonate, besides bringing the pH of the reaction solution to the desired value, is also particularly effective at forming *in situ* microporosities in the CMC mass during its crosslinking, thanks to the development of gaseous $CO_2$ following the reaction between boric acid and sodium hydrogen carbonate.

In one embodiment, the method could provide a preliminary step to step b) wherein the solution is subjected to a step of pre-evaporation; the step of pre-evaporation is performed, e.g., at a temperature between room temperature and 45°C, for a time ranging, e.g., between 48 and 120 hours.

In one embodiment, the method provides the dissolution of boric acid in water in a concentration equal to 4% of the CMCNa; the boric acid solution initially has a pH equal to 5.6; the addition of an alkalinizing agent, for instance diluted solutions of NaOH or $NaHCO_3$, leads to have a solution at a pH between 8 and 9. Then, sucrose is solubilized in a concentration equal to 4% by weight of water, and CMCNa is slowly added until having a CMCNa solution equal to minimum 2% w/w of the solvent.

After a homogenizing stage at room temperature under mechanical or magnetic stirring, one can carry on by following two routes:

> 1) first a pre-evaporation stage, e.g. in an oven at 45°C for at least 48 hours, followed by a crosslinking stage at 80°C for times ranging from 0 to 7 hours;
> 2) crosslinking reaction, without pre-evaporation, in an oven at 80°C for times ranging between 3 and 7 hours.

In another embodiment, solubilization is performed in a suitable vessel, containing water from the (optional) spacer, followed by slow addition of CMCNa. In another vessel, boric acid and/or boronic acids and/or borax is/are solubilized in water (in concentrations between 2 and 8%), increasing the pH of said solution with a sodium-based basic substance. The resulting solution of sodium borate could be, e.g., added dropwise in the polysaccharide solution under stirring. Then, one can proceed with first a pre-evaporation stage, e.g. in an oven at 45°C for at least 48 hours, followed by a crosslinking stage at 80°C for times ranging between 3 and 7 hours.

The method of the present invention provides the drying of the gel obtained at step b), said step of drying is performed, e.g., at at least 45°C for variable times, depending on the thickness that is to be obtained (preferably from a minimum of 48 to a maximum of 120 hours). For the drying, conventional techniques such as air-drying or oven-drying could be used. Before the drying step, one or more stages of gel washing, e.g. with water, could optionally be provided.

The method of the present invention could provide a further step d) wherein the dried polymer is reduced as powder, e.g. by milling, obtaining small-sized particles (e.g., between 300 and 600 $\mu$m) after optional selection by sieving.

The polymers of the present invention find various applications; for instance, they could be utilized in pharmaceutical compositions for the controlled release of active principles contained in said pharmaceutical compositions.

The experiments reported in the present description indicate that the polymers described herein, besides having a very high absorbent power when measured over short kinetics, also have a high absorption power over very long kinetics.

This property makes the polymers of the present invention particularly suitable to be utilized in the absorbent core of absorbent products for absorbing body fluids, such as diapers, feminine towels, gauzes, bandages.

The powders obtained according to the method described in the present invention find further applications in the following sectors:

- absorbent products for food packaging (absorbent pads for foods) such as the making of mats that may absorb meat and fish exudates in packagings, or the making of absorbent mats for fruit and vegetable transport.
- agricultural products (systems for controlled release of water or nutrients for arid soils, etc.) - The mixing of biodegradable hydrogels, optionally charged with nutrient products, with soils can allow, once watered and swollen, greater oxygenation of the roots, besides releasing water over long periods of time in a controlled way.

Methods utilized to assess hydrogels performance

Assessment of hydrogels performance was carried out through the following tests:

> 1) swelling in saline solution (0.9% NaCl) of 0.1 g of dry product for 5 minutes in "tea bag" (i.e., polyester bag) followed by centrifugation (in order to obtain the centrifugation retention capacity, CRC) at 1400 rpm for 3 minutes (EDANA method 441.2-02).
> CRC measures SAP capacity to retain liquid thereinside after having been saturated and subjected to centrifugation under controlled conditions. It is expressed in grams of water over grams of dry sample.

The data obtained from said test are:

CRC = [(hydrated tea bag weight after centrifuge) - (tea bag+dry polymer weight)]/(dry polymer weight)

2) swelling in saline solution (0.9% NaCl) of 0.5 g of dry product for 15 minutes, followed by draining of liquid not retained by the gel into sintered glass funnels for 5 minutes (free swell measurement) and subsequent draining of liquid not retained by the gel under action of a 3 Kpa (0.435 PSI) load, following Technical Absorbents Standard Test Method No. 2. By putting the load on the hydrated gel, retention under load of the sap can be estimated. The data obtained from said test are:

*free swell* = [(polymer+saline solution weight)-(freely drained saline solution weight after 5')]/(dry polymer weight)

retention under load = [(polymer+saline solution weight)-(weight of freely drained saline solution after a 5' applying of the weight)]/(dry polymer weight)

3) absorption under load (AUL), evaluated on 0.3 g of dry product for 30 minutes under a 0.14 psi load.

The test measures SAP ability to absorb the saline solution (0.9% NaCl) against a pressure that varies depending on the type of employ of the SAP. In said test, SAP is placed inside a plastics cylinder, at the base of which there is a 200-$\mu$m filter. The weight realizing the desired pressure is placed thereon and left there for the desired time. Absorbency under load is determined in g/g. The test principle is described in European standard EDANA ERT 442 - Gravimetric Determination of Absorption under Pressure or Absorbency Under Load.

Examples aimed at better illustrating the properties of the hydrogels of the present invention and some specific embodiments thereof are reported hereinafter; in no way such examples are to be construed as a limitation of the preceding description and of the claims hereinafter.

EXAMPLE 1

CMCNa crosslinking by boric acid without pre-evaporation

CMCNa (commercially available as 7H4F from Eigenmann & Veronelli SpA, Milan, Italy) and boric acid from Sigma Aldrich, Italy, were employed without any previous treatment.

Hydrogel synthesis

CMCNa was dissolved in an aqueous solution, alkalinized with NaOH to pH 8-9, containing boric acid, 4% of CMCNa. In said configuration, no spacer was employed. CMCNa concentration is equal to 2% of the solvent. CMCNa dissolution is slow and requires at least 24 hours of homogenizing under constant mechanical or magnetic stirring.

Solution viscosity is initially very high. Then, crosslinking is directly performed, under constant stirring, for 5 hours at 80°C.

The material is then dried in a laboratory stove at 45°C for 48 hours, milled and sieved (in the 300-600 $\mu$m range), stored tel quel (as is) or subjected to successive washing and drying in two modes: by oven drying (in an oven at 45°C) or by phase inversion in a non-solvent (acetone).

Characterization:

Surface analysis by X-ray Photoelectronic Spectroscopy (XPS):

XPS measurements were performed by using a ThermoVG Theta probe spectrometer, with a monochromatic Al K$\alpha$ source (1486.6 eV). For each sample, wide scan spectra (BE ranging between 0 and 1200 eV, fixed analyser transmission mode, step energy = 150 eV) and high resolution spectra (fixed analyser transmission mode, step energy = 50 eV) were recorded. High-resolution spectra were processed for signal deconvolution by utilizing Avantage software, which consists in a spectral fitting program based on a non-linear method of least squares. In all cases, peaks with Lorentzian/Gaussian ratios and mid-height amplitude equal inside a same signal were employed, whereas quantitation was performed by employing normalized peak areas (i.e., divided for empirical sensitivity factors).

Fourier Transform Infrared Analysis (FT-IR) in Attenuated Total Reflectance (ATR) mode:

FT-IR measurements were performed by PerkinElmer Spectrum Two spectrophotometer, with an optical system with KBr windows collecting data over a 7800-525 cm$^{-1}$ range, with a maximum resolution of 0.5 cm$^{-1}$; the detector is a LiTaO$_3$ detector for mid-IR (MIR) and works at room temperature. This spectrophotometer can work in transmittance but it also provides ATR configuration, by the Universal ATR *(Single Reflection Diamond)* tool for solid and liquid analysis, without any previous preparative stage.

Thermal analysis by Differential Scanning Calorimetry (DSC):

DSC measurements were performed by Perkin Elmer DSC400, equipped with Pyris software for thermogram processing.

Two typologies of analysis were performed: one in which the temperature range explored was 30-150°C, at 10°C/minute; the second one in which the following thermal program was employed: (1) 30-100°C heating at 10°C/min; (2) 100°C isotherm for 3 minutes; (3) 100-30°C cooling at 10°C/min; (4) 30-200°C heating at 10°C/min; (5) 200°C isotherm for 3 minutes; (6) 100-30°C cooling at 10°C/min.

Thermal analysis by Thermogravimetry (TGA):

Thermogravimetric measurements were performed by TGA TA-Instrument Q500, div. Di Waters S.p.A., Milan, Italy, under air atmosphere, according to the following thermal program: stabilization at 40°C, 40-1000°C isotherm at 10°C/minute.

Results and Discussion

XPS surface analysis enabled to single out the presence of surface Boron in the hydrogel not subjected to any treatment successive to crosslinking, in atomic percentages ranging from 1 to 1.5%, besides the presence of Carbon, Oxygen and Sodium signals attributable to the starting CMCNa.

By FT-IR analysis in ATR mode it was possible to compare the absorption spectrum of the starting CMCNa, of sodium borate, and that of the hydrogel synthesized from boric acid. It is observed that, since there are no sodium borate absorption bands in zones where there are no CMCNa absorptions, it was necessary to assess the height increase of an absorption peak (in particular that of sodium borate at 1414.9 cm$^{-1}$) relative to that typical of CMCNa (at about 1590 cm$^{-1}$) through the ratio of the areas of such peaks, over the absorbance peaks of all three samples under examination. Note that in the spectrum of hydrogel 1_l (taken as example) it is had an increase of about 15% of such ratio of areas compared to CMCNa, which is probable evidence of the presence of absorptions due to the B-OR bond in the hydrogel.

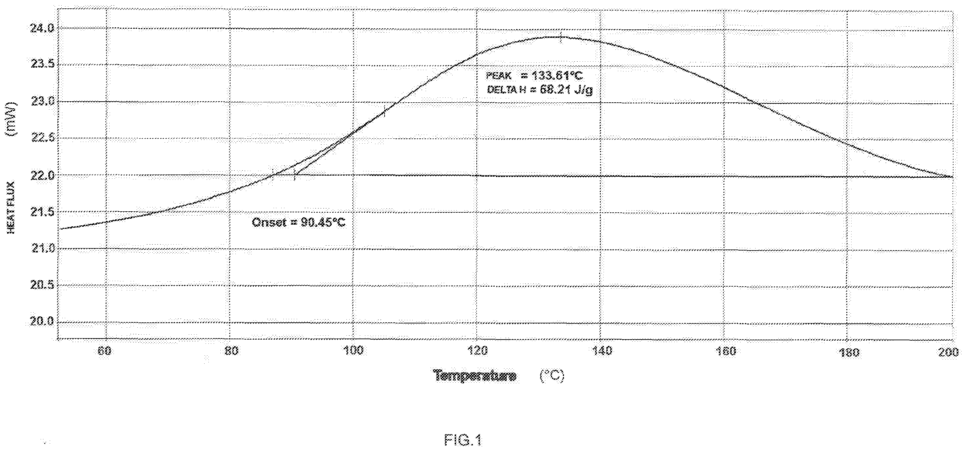

Starting CMCNa was analyzed beforehand by DSC analysis, in order to assess the temperature range within which to perform the crosslinking reaction. By analyzing in particular the second heating, an endothermal event centered at about 134°C is observed, with onset at about 90°C, probably linked to CMCNa decomposition (FIG.1). Finally, there was performed a thermogravimetric analysis on the hydrogel tel quel (1_tq) and of the same after drying process by phase inversion in acetone (1_if).

The aim is that of detecting the moisture content in the powders analyzed and, secondly, of obtaining information on their thermal stability (that is, understanding at what temperatures a significant degradation of the material starts in the presence of an oxidizing gas such as air).

Moisture and/or volatile substances content in the two samples (i.e., the powder tel quel and that after washing and phase inversion) is practically the same (about 5.7%). First degradation of the material is observed at about 230°C, therefore the end material proves to be rather stable, even under oxidizing conditions.

Residue at 1000°C proves to be still very high in both samples analyzed, a data attributable also to the presence of Boron-based inorganic material, and it is about 10%.

Liquid absorption and retention data

The data of retention after centrifugation, related to the swelling in aqueous solution of 0.1 g of dry product for 5 minutes in a "tea bag", are reported in the table:

| Product code | washing | Drying by phase inversion | Retention after centrifugation |
| --- | --- | --- | --- |
| 1_tq | no | no | 31 |

(continued)

| Product code | washing | Drying by phase inversion | Retention after centrifugation |
|---|---|---|---|
| 1_l | yes | no | 31 |
| 1_if | yes | yes | 23 |

The data of free absorption for 15 minutes and retention under load for 5 minutes (3 Kpa - 0.435 PSI) in saline solution (0.9% NaCl) by 0.5 g of dry product are reported in the following table:

| Product code | Washing | Drying by phase inversion | Free swell | Retention under load |
|---|---|---|---|---|
| 1_tq | no | no | 41 | 40 |
| 1_l | yes | no | 45 | 44 |
| 1_if | yes | yes | 35 | 16 |

The data of absorption, under a load of 0.14 psi, of 0.3 g of dry product for 30 minutes in saline solution, are reported in the following table:

| Product code | Washing | Drying by phase inversion | Absorption under load |
|---|---|---|---|
| 1_tq | no | no | 16 |
| 1_l | yes | no | 15 |
| 1_if | yes | yes | 15 |

Conclusions

This example highlights fair end performances of the CMCNa-based hydrogel, crosslinked with boric acid and obtained without employing an intermediate step of pre-evaporation.

In particular, the polymer tel quel and that washed in water and stove-dried exhibit interesting absorbent properties, releasing reasonable amounts of liquids under loading conditions. Also the test with the centrifugation highlighted properties of retaining absorbed liquid amounts comparable to commercial SAPs.

The use of phase inversion as drying procedure leads to a gel that is less performing in terms of free swelling and CRC and does not afford improvements in terms of absorption under load.

Example 2

CMCNa crosslinking by boric acid with pre-evaporation

CMCNa (commercially available as 7H4F from Eigenmann & Veronelli SpA, Milan, Italy) and boric acid from Sigma Aldrich, Italy, were employed without any previous treatment.

Hydrogel synthesis

CMCNa was dissolved in an aqueous solution, alkalinized with 0.5M NaOH to pH 8-9, containing boric acid, at 4% of CMCNa. In said configuration, no spacer was employed.

CMCNa concentration is equal to 2% of the solvent. CMCNa dissolution is slow and requires at least 24 hours of homogenizing under constant mechanical or magnetic stirring.

Solution viscosity is initially very high.

After said homogenizing stage, the material is transferred into aluminum or Teflon pans in order to obtain thin films, on which a pre-evaporation of water contained therein is performed.

Said step is performed in an oven at 45 °C for minimum 48 and maximum 120 hours, followed by a crosslinking stage at 80°C for 7 hours.

Characterization:

The hydrogel synthesized in this configuration was characterized by analytical techniques reported in example 1. Both from a chemical and thermal standpoint, no significant differences with the material of example 1 are observed.

Liquid absorption and retention data

The data of retention after centrifugation, related to the swelling in aqueous solution of 0.1 g of dry product, are reported in the table:

| Product code | washing | Drying by phase inversion | Retention after centrifugation |
|---|---|---|---|
| 2_tq | no | no | 33 |
| 2_l | yes | no | 31 |
| 2_if | yes | yes | 20 |

The data of free absorption for 15 minutes and retention under load for 5 minutes (3 Kpa - 0.435 PSI) in saline solution (0.9% NaCl) by 0.5 g of dry product are reported in the following table:

| Product code | Washing | Drying by phase inversion | Free swell | Retention under load |
|---|---|---|---|---|
| 2_tq | no | no | 50 | 50 |
| 2_l | yes | no | 44 | 43 |
| 2_if | yes | yes | 25 | 23 |

The data of absorption under a load of 0.14 psi of 0.3 g of dry product for 30 minutes in saline solution are reported in the following table:

| Product code | Washing | Drying by phase inversion | Absorption under load |
|---|---|---|---|
| 2_tq | no | no | 15 |
| 2_l | yes | no | 15 |
| 2_if | yes | yes | 16 |

Conclusions

This example highlights the effective advantage in terms of end performance of the CMCNa-based hydrogel, crosslinked with boric acid and obtained by employing an intermediate step of pre-evaporation.

In particular, the polymer tel quel and, to follow, that washed in water and stove-dried, exhibit surprising absorbent properties, releasing extremely reasonable amounts of liquids under loading conditions, yielding results even superior to those of commercial SAPs tested under the same conditions. Also the test with centrifugation, performed in particular on the material tel quel, i.e., not subjected to any additional washing and drying, highlighted properties of retaining amounts of absorbed liquids that are comparable to commercial SAPs. As in example 1, there are no particular advantages in the use of phase inversion as drying procedure, even in the case of absorption tests under load. In fact, the material tel quel, or the same after washing and stove drying, yielded the most interesting results.

Example 3

CMCNa crosslinking by boric acid in the presence of sucrose spacer without pre-evaporation

CMCNa (commercially available as 7H4F from Eigenmann & Veronelli SpA, Milan, Italy, boric acid and sucrose from Sigma Aldrich, Italy, were employed without any preliminary treatment.

Hydrogel synthesis

Sucrose and CMCNa (respectively, 4 and 2% of the solvent) were dissolved in an aqueous solution, alkalinized with NaOH 0,5M to pH 8-9, containing boric acid, 4% of CMCNa. In said configuration, no spacer was employed.

While sucrose dissolution is rather quick, that of CMCNa is slow and requires at least 24 hours of homogenizing under constant mechanical or magnetic stirring.

Solution viscosity is initially very high. Then, crosslinking is directly performed, under constant stirring, for 5 hours at 80°C.

The material is then dried in a laboratory stove at 45°C for 48 hours, milled and sieved (in the 300-600 $\mu$m range), stored tel quel or subjected to subsequent washing and drying in two modes: by oven drying (in an oven at 45 °C) or by phase inversion in a non-solvent (acetone).

Characterization

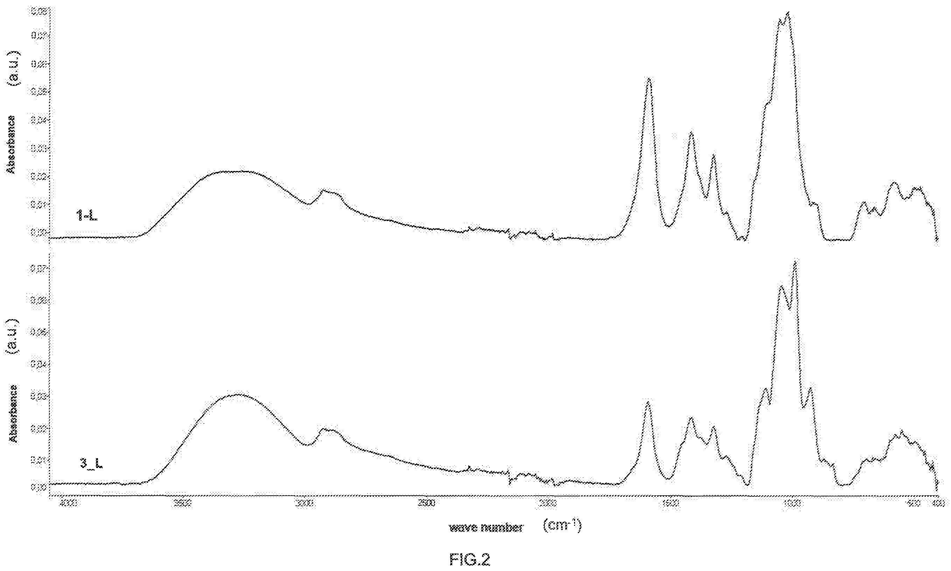

The hydrogel synthesized in this configuration was characterized by analytical techniques reported in example 1. From a chemical standpoint, there are observed differences linked to the presence of sucrose as molecular spacer in the hydrogel relative to this example, highlighted in particular by ATR analysis (FIG.2). In said figure, the FT-IR spectrum in ATR mode of a CMCNa-based hydrogel, crosslinked with boric acid with (1_l) and without (3_l) sucrose, after washing, is reported. Note, in particular, the zone between 700 and 1200 cm$^{-1}$, in which sucrose presence leads to a different form of such absorption bands, compared to the sole CMCNa-based material, confirming the fact that the spacer, even after washing the sucrose is present in the macromolecules, as covalently bound to the polymeric network of CMCNa by the crosslinking agent.

Liquid absorption and retention data

The data of retention after centrifugation, related to the swelling in aqueous solution of 0.1 g of dry product for 5 minutes in a "tea bag", are reported in the table:

| Product code | washing | Drying by phase inversion | Retention after centrifugation |
|---|---|---|---|
| 3_tq | no | no | 26 |
| 3_l | yes | no | 23 |
| 3_if | yes | yes | 22 |

The data of free absorption for 15 minutes and retention under load for 5 minutes (3 Kpa - 0.435 PSI) in saline solution (0.9% NaCl) by 0.5 g of dry product are reported in the following table:

| Product code | Washing | Drying by phase inversion | Free swell | Retention under load |
|---|---|---|---|---|
| 3_tq | no | no | 44 | 18 |
| 3_l | yes | no | 38 | 17 |
| 3_if | yes | yes | 39 | 16 |

Data of absorption, under a load of 0.14 psi, of 0.3 g of dry product for 30 minutes in saline solution, are reported in the following table:

| Product code | Washing | Drying by phase inversion | Absorption under load |
|---|---|---|---|
| 3_tq | no | no | 12 |
| 3_l | yes | no | 10 |
| 3_if | yes | yes | 12 |

Conclusions

This example highlights that the employ of a spacer such as sucrose leads to have a hydrogel with performance inferior to that reported in the example 1.

Therefore, it is assumed that, in order to increase liquid absorption and retention, the employ of substrates different from CMCNa (e.g., CMCNa-amid, etc.) or spacers different from sucrose (e.g., monosaccharides, such as fructose, or polyalcohols, such as sorbitol etc.) be desirable, varying also their relative percentages or hydrogel synthesis conditions.

Example 4

CMCNa crosslinking by boric acid in the presence of sucrose spacer and with pre-evaporation

CMCNa (commercially available as 7H4F from Eigenmann & Veronelli SpA, Milan, Italy, sucrose and boric acid from Sigma Aldrich, Italy, were employed without any previous treatment.

Hydrogel synthesis

Sucrose and CMCNa (respectively 4 and 2% of the solvent) were dissolved in an aqueous solution, alkalinized with 0.5M NaOH to pH 8-9, containing boric acid, 4% of CMCNa. In said configuration, no spacer was employed.
While sucrose dissolution is rather quick, that of CMCNa is slow and requires at least 24 hours of homogenizing under constant mechanical or magnetic stirring.
After said homogenizing stage, the material is transferred into aluminum or Teflon pans in order to obtain thin films, on which a pre-evaporation of water contained therein is performed.
Said step is performed in an oven at 45 °C for minimum 48 and maximum 120 hours, followed by a crosslinking stage at 80°C for 7 hours.

Characterization

The hydrogel synthesized in this configuration was characterized by analytical techniques reported in example 1. Both from a chemical and thermal standpoint, no significant differences with the material of Example 3 are observed.

Liquid absorption and retention data

The data of retention after centrifugation, related to the swelling in aqueous solution of 0.1 g of dry product for 5 minutes in a "tea bag", are reported in the table:

| Product code | Washing | Drying by phase inversion | Retention after centrifugation |
| --- | --- | --- | --- |
| 4_tq | no | no | 31 |
| 4_l | yes | no | 27 |
| 4_if | yes | yes | 24 |

The data of free absorption for 15 minutes and retention under load for 5 minutes (3 Kpa - 0.435 PSI) in saline solution (0.9% NaCl) by 0.5 g of dry product are reported in the following table:

| Product code | Washing | Drying by phase inversion | Free swell | Retention under load |
| --- | --- | --- | --- | --- |
| 4_tq | no | no | 40 | 35 |
| 4_l | yes | no | 45 | 39 |
| 4_if | yes | yes | 44 | 24 |

The data of absorption, under a load of 0.14 psi, of 0.3 g of dry product for 30 minutes in saline solution, are reported in the following table:

| Product code | Washing | Drying by phase inversion | Absorption under load |
|---|---|---|---|
| 4_tq | no | no | 12 |
| 4_l | yes | no | 13 |
| 4_if | yes | yes | 13 |

Conclusions

This example highlights that the CMCNa- and sucrose-based hydrogel, crosslinked with boric acid and obtained by employing an intermediate step of pre-evaporation, gives superior results of absorption and retention under load or after centrifugation, compared to results obtained in Example 3, where the pre-evaporation step is not present, making the Inventors conclude that such step is particularly useful when a spacer is utilized in the gel formulation. Finally, like for the preceding examples, there are no particular advantages in the use of phase inversion as drying procedure, as also emerging from the test of absorption under load.

Example 5

CMCNa crosslinking by boric acid and sodium hydrogen carbonate with pre-evaporation

CMCNa (commercially available as 7H4F from Eigenmann & Veronelli SpA, Milan) and boric acid from Sigma Aldrich, Italy, were employed without any previous treatment.

Hydrogel synthesis

$NaHCO_3$ was dissolved in a distilled water, until obtaining a pH greater than 8. In said solution, boric acid was dissolved in a concentration such as to be 4% of CMCNa. The pH of the solution after boric acid addition did not vary noticeably. CMCNa was dissolved in said solution.
CMCNa concentration was equal to 2% of the solvent. CMCNa dissolution is slow and requires at least 24 hours of homogenizing under constant mechanical or magnetic stirring.
Solution viscosity is very high.
After said homogenizing stage, the material is transferred into aluminum or Teflon pans in order to obtain thin films, on which a pre-evaporation of water contained therein is performed.
Said step is performed in a stove at 45°C for minimum 48 and maximum 120 hours, followed by a crosslinking stage at 80°C for 0, 3 or 7 hours.

Characterization:

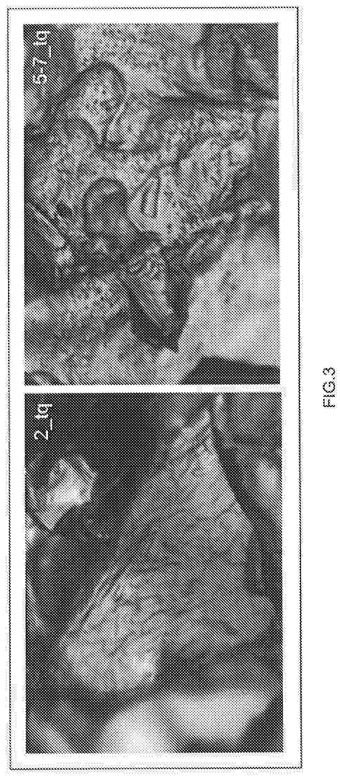

The hydrogel synthesized in this configuration was characterized by analytical techniques reported in example 1. Both from a chemical and thermal standpoint, no significant differences with the material of Example 2 are observed.
From a morphological standpoint, instead, differences are observed between the material obtained by employing NaOH (2_tq) and that obtained by employing $NaHCO_3$ (5-7_tq) (FIG.3). It is interesting to note that, while the gel from NaOH has a smoother structure, the gel from $NaHCO_3$ shows a series of small diffuse porosities, probably correlatable to the fact that said hydrogel was synthesized in the presence of sodium hydrogen carbonate that, by reacting with boric acid to give the borate, transforms into $CO_2(g)$, evolving inside the gel being formed, through gaseous microbubbles.

Liquid absorption and retention data

The data of retention after centrifugation, related to the swelling in aqueous solution of 0.1 g of dry product, are reported in the table below. Having assessed beforehand that washings and drying by phase inversion in acetone afforded no improvements to material performance, said material was tested tel quel. What was taken into account was the crosslinking times, as reported in the following Table:

| Product code | Crosslinking time | Retention after centrifugation |
|---|---|---|
| 5-0_tq | 0 | 27 |

(continued)

| Product code | Crosslinking time | Retention after centrifugation |
|---|---|---|
| 5-3_tq | 3 | 31 |
| 5-7_tq | 7 | 35 |

The data of free absorption for 15 minutes and retention under load for 5 minutes (3 Kpa - 0.435 PSI) in saline solution (0.9% NaCl) by 0.5 g of product are reported in the following Table:

| Product code | Crosslinking time | Free swell | Retention under load |
|---|---|---|---|
| 5-0_tq | 0 | 44 | 40 |
| 5-3_tq | 3 | 51 | 49 |
| 5-7_tq | 7 | 51 | 50 |

The data of absorption, under a load of 0.14 psi, of 0.3 g of dry product for 30 minutes in saline solution, are reported in the following table:

| Product code | Washing | Drying by phase inversion | Absorption under load |
|---|---|---|---|
| 5-0_tq | no | no | 16 |
| 5-3_tq | yes | no | 18 |
| 5-7_tq | yes | yes | 18 |

Conclusions

The hydrogel was obtained by employing an intermediate step of pre-evaporation, followed by a subsequent step of crosslinking, whose time ranged between 0, 3 and 7 hours.

It is interesting to note that, from the results obtained on the material, it is inferred that crosslinking probably starts already in the pre-evaporation stage at 45°C. In case of the tests of free absorption, retention under load and absorption under load, an improvement is observed after 3 hours of crosslinking at 80°C. No drastic improvements are observed by prolonging the crosslinking times, as an extremely convincing result is obtained already at +3 hours.

Example 6

CMCNa crosslinking by boric acid at different concentrations and sodium hydrogen carbonate with pre-evaporation

CMCNa (commercially available as 7H4F from Eigenmann & Veronelli SpA, Milan, Italy) and boric acid from Sigma Aldrich, Italy, were employed without any previous treatment.

Hydrogel synthesis

$NaHCO_3$ was dissolved in a distilled water, until obtaining a pH greater than 8. In said solution, boric acid was dissolved in concentration between 2 and 8% of the CMCNa. The pH of the solution after boric acid addition did not vary noticeably. CMCNa was dissolved in said solution.

CMCNa concentration was equal to 2% of the solvent. CMCNa dissolution is slow and requires at least 24 hours of homogenizing under constant mechanical or magnetic stirring.

Solution viscosity is very high.

After said homogenizing stage, the material is transferred into aluminum or Teflon pans in order to obtain thin films, on which a pre-evaporation of water contained therein is performed.

Said step is performed in an oven at 45°C for minimum 48 and maximum 120 hours, followed by a crosslinking stage at 80°C for 3 or 7 hours.

Liquid absorption and retention data

The data of retention after centrifugation related to the swelling in aqueous solution of 0.1 g of dry product, obtained by employing 2, 6 and 8% boric acid, with crosslinking times of 3 and 7 hours, are reported in the table:

| Product code | Crosslinking time | Retention after centrifugation |
|---|---|---|
| 6-2%_tq | 3 | 16 |
| 6-2%_tq | 7 | 32 |
| 6-6%_tq | 3 | 18 |
| 6-6%_tq | 7 | 23 |
| 6-8%_tq | 3 | 24 |
| 6-8%_tq | 7 | 25 |

The data of free absorption for 15 minutes and retention under load for 5 minutes (3 Kpa - 0.435 PSI) in saline solution (0.9% NaCl) by 0.5 g of product are reported in the following table:

| Product code | Crosslinking time | Free swell | Retention under load |
|---|---|---|---|
| 6-2%_tq | 3 | 51 | 40 |
| 6-2%_tq | 7 | 49 | 40 |
| 6-6%_tq | 3 | 51 | 47 |
| 6-6%_tq | 7 | 51 | 50 |
| 6-8%_tq | 3 | 51 | 48 |
| 6-8%_tq | 7 | 52 | 52 |

The data of absorption, under a load of 0.14 psi, of 0.3 g of dry product for 30 minutes in saline solution, are reported in the following table:

| Product code | Crosslinking time | Absorption under load |
|---|---|---|
| 6-2%_tq | 3 | 15 |
| 6-2%_tq | 7 | 16 |
| 6-6%_tq | 3 | 13 |
| 6-6%_tq | 7 | 13 |
| 6-8%_tq | 3 | 12 |
| 6-8%_tq | 7 | 14 |

Conclusions

This example highlights the dependence of hydrogel features on (1) concentration of boric acid employed, and (2) crosslinking times.

In particular, it may be inferred that there are different responses based on the test employed and, in particular:

as to retention after centrifugation, the best results are observed for hydrogels having low concentrations of crosslinking agent ($\leq 4\%$), as expected, since it is known that by decreasing the degree of crosslinking an increase of CRC is observed;

as to absorption and retention under load, the results obtained would lead to favor boric acid concentrations of $\geq$ 4%, probably because gels more crosslinked, i.e. with a thicker packing of the polymer chains, are able to more readily endure the load to which they are subjected, expelling less liquid (in particular, the material obtained by employing an initial baric acid concentration of 8% does not release liquid at all during the test of saline solution retention under load);

as to absorption under load, a greater crosslinking did not lead to improvements. An increase of crosslinking agent concentration to > 4% values does not foster the formation of a stiffer gel, able under load to swell by taking on liquid.

Anyway, it may be inferred from what reported in the foregoing that boric acid concentrations of 4%, with minimum crosslinking times of 3 hours, prove to be effective in order to obtain a carboxymethylcellulose-based superabsorbent system capable of satisfying the required functional requirements, i.e. high liquid absorption rates and low or nil liquid release both under conditions of inertia, and of mobility as well as of load.

Example 7

Crosslinking of the CMCNa/HEC mixture by boric acid with pre-evaporation

CMCNa (commercially available as 7H4F from Eigenmann & Veronelli SpA, Milan, Italy), hydroxyethylcellulose (HEC) (commercially available as Natrosol 250M from Eigenmann & Veronelli SpA, Milan, Italy), were employed without any previous treatment.

Hydrogel synthesis

CMCNa and HEC (respectively, 1.5 and 0.5% of the solvent) were dissolved in an aqueous solution, alkalinized with 0.5M NaOH to pH 8-9, containing boric acid, at 4% of the precursors (CMCNa+HEC). In said configuration, no spacer was employed. CMCNa dissolution in the presence of HEC is slow and requires at least 24 hours of homogenizing under constant mechanical or magnetic stirring.

After said homogenizing stage, the material is transferred into aluminum or Teflon pans in order to obtain thin films, on which a pre-evaporation of water contained therein is performed.

Said step is performed in an oven at 45°C for minimum 48 and maximum 120 hours, followed by a crosslinking stage at 80°C for 0, 3 or 7 hours. The product tel quel, i.e. not subjected to washings or further treatments, was subsequently milled and sieved in order to obtain particles of the order of 300-600 $\mu$m.

Characterization

The hydrogel synthesized in this configuration was characterized by analytical techniques reported in example 1. It is observed in particular an increase of the peaks attributed to the symmetrical stretching of the $CH_2$ at about 2876 $cm^{-1}$ and of the scissoring vibrations of the same group, at about 1455 $cm^{-1}$, increase due to HEC presence. Also a light-intensity peak can be observed, very intense in the spectrum of pure HEC, at 1647 $cm^{-1}$, probably attributable to the bending of naturally-adsorbed water molecules.

Liquid absorption and retention data

The data of retention after centrifugation, related to the swelling in aqueous solution of 0.1 g of dry product, are reported in the table below. Having assessed beforehand that washings and drying by phase inversion in acetone afforded no improvements to material performance, said material was tested tel quel. What was taken into account was the crosslinking times, as reported in the following Table:

| Product code | Crosslinking time | Retention after centrifugation |
|---|---|---|
| 7-0_tq | 0 | 17 |
| 7-3_tq | 3 | 20 |
| 7-7_tq | 7 | 20 |

The data of free absorption for 15 minutes and retention under load for 5 minutes (3 Kpa - 0.435 PSI) in saline solution (0.9% NaCl) by 0.5 g of product are reported in the following table:

| Product code | Crosslinking time | Free swell | Retention under load |
|---|---|---|---|
| 7-0_tq | 0 | 45 | 41 |

(continued)

| Product code | Crosslinking time | Free swell | Retention under load |
|---|---|---|---|
| 7-3_tq | 3 | 49 | 48 |
| 7-7_tq | 7 | 46 | 46 |

The data of absorption, under a load of 0.14 psi, of 0.3 g of dry product for 30 minutes in saline solution, are reported in the following table:

| Product code | Crosslinking time | Absorption under load |
|---|---|---|
| 7-0_tq | 0 | 10 |
| 7-3_tq | 3 | 13 |
| 7-7_tq | 7 | 14 |

Conclusions

This example highlights that the hydrogel based on CMCNa and HEC crosslinked with boric acid employing an intermediate step of pre-evaporation gives results of absorption and retention under load, or after centrifugation, inferior to those related to hydrogels from CMCNa alone.

In this case, as in Example 5, the crosslinking probably starts already in the pre-evaporation stage at 45°C and can be considered complete already after 3 hours of reaction at 80°C, as is inferred also by the results of the test of absorption under load.

Example 8

CMCNa crosslinking alone, or in mixture with HEC, in the presence of bentonite filler by boric acid with pre-evaporation

CMCNa (commercially available as 7H4F from Eigenmann & Veronelli SpA, Milan, Italy) alone or in mixture with HEC (commercially available as Natrosol 250M from Eigenmann & Veronelli SpA, Milan, Italy), Sodium bentonite (commercially available as AG/8W from Dal Cin SpA, Italy), were employed without any previous treatment.

Hydrogel synthesis

CMCNa alone or in mixture with HEC (respectively, 2% and/or 1.5+0.5% of the solvent) and Bentonite (0.5% of the solvent) were premixed dry and subsequently dissolved in an aqueous solution, alkalinized with $NaHCO_3$ at pH 8-9, containing boric acid, 4% of CMCNa or of CMCNa/HEC. In said configuration, no spacer was employed.

CMCNa/bentonite mixture dissolution is slow and requires at least 24 hours of homogenizing under constant mechanical or magnetic stirring.

After said homogenizing stage, the material is transferred into aluminum or Teflon pans in order to obtain thin films, on which a pre-evaporation of water contained therein is performed.

Said step is performed in an oven at 45 °C for minimum 48 and maximum 120 hours, followed by a crosslinking stage at 80°C for 7 hours. The product tel quel, i.e., not subjected to washings or further treatments, was subsequently milled and sieved in order to obtain particles of the order of 300-600 μm.

Characterization

The hydrogel synthesized in this configuration was characterized by analytical techniques reported in example 1.

The FT-IR spectrum of pure bentonite was acquired beforehand, in order to assess any additional contributions attributable thereto in samples 8_tq and 8H_tq. In particular, in the bentonite spectrum are observed: a small absorption band at about 3615 $cm^{-1}$, which is attributed to the stretching of OH that are present in the smectite, a band at about 1635 $cm^{-1}$, attributable to bending of the Si-OH group, and a band at about 1000 $cm^{-1}$, assignable to the stretching of the Si-O group, and at about 910 $cm^{-1}$, to vibrations of the Al-OH groups. Instead, it is not observed the presence of a band at about 1430 $cm^{-1}$, due to calcite impurities. In samples 8_tq and 8H_tq an absorption increase is observed at about 1008 $cm^{-1}$, already present in cellulose derivatives, that can be due to intense absorption of bentonite Si-OH groups.

Liquid absorption and retention data

The data of retention after centrifugation, related to the swelling in aqueous solution of 0.1 g of dry product, are reported in the table below. Having assessed beforehand that the washings and drying by phase inversion in acetone afforded no improvements to material performance, said material was tested tel quel. What was assessed was the employ of CMCNa, alone or in mixture with HEC, in both cases having employed the bentonite filler at the same concentration, as reported in the following Table.

| Product code | substrate | Retention after centrifugation |
|---|---|---|
| 8_tq | CMCNa | 33 |
| 8-H_tq | CMCNa/HEC | 20 |

The data of free absorption for 15 minutes and retention under load for 5 minutes (3 Kpa - 0.435 PSI) in saline solution (0.9% NaCl) by 0.5 g of product are reported in the following table:

| Product code | substrate | Free swell | Retention under load |
|---|---|---|---|
| 8_tq | CMCNa | 50 | 49 |
| 8-H_tq | CMCNa/HEC | 43 | 41 |

The data of absorption, under a load of 0.14 psi, of 0.3 g of dry product for 30 minutes in saline solution, are reported in the following table:

| Product code | substrate | Absorption under load |
|---|---|---|
| 8_tq | CMCNa | 19 |
| 8-H_tq | CMCNa/HEC | 14 |

Conclusions

This example highlights that the addition of an inorganic filler such as bentonite affords substantial improvements to the performance of materials obtained from CMCNa alone; on the contrary, in the CMCNa/HEC combination the addition of Bentonite does not entail significant variations.

Example 9

CMCNa crosslinking in the presence of laponite or bentonite + laponite filler, by boric acid with pre-evaporation

CMCNa (commercially available as 7H4F from Eigenmann & Veronelli SpA, Milan, Italy), Laponite™ (commercially available as Laponite®, RDS Rockwood Additives Limited: A Rockwood Holdings, Inc. Company, UK) and Sodium bentonite (commercially available as AG/8W from Dal Cin SpA, Italy) were employed without any previous treatment.

Hydrogel synthesis

CMCNa (2% of the solvent) alone or in mixture with Sodium bentonite (0.5% of the solvent) was dissolved in an aqueous solution, alkalinized with $NaHCO_3$ to pH 8-9, containing boric acid, 4% of CMCNa. CMCNa or CMCNa/bentonite mixture dissolution is slow and requires at least 24 hours of homogenizing under constant mechanical or magnetic stirring. In said configuration, no spacer was employed.
Laponite™ (0.1% of total solvent amount) was previously dissolved in the minimum amount of solvent recommended by the specifications, in order to obtain complete solubilization thereof (requiring maximum times of 1 hour), to be then additioned to the aqueous solution of CMCNa or to that of CMCNa/Bentonite.
After said homogenizing stage, the material is transferred into aluminum or Teflon pans in order to obtain thin films, on which a pre-evaporation of water contained therein is performed.
Said step is performed in an oven at 45 °C for minimum 48 and maximum 120 hours, followed by a crosslinking stage at 80°C for 7 hours. The product tel quel, i.e. not subjected to washings or further treatments, was subsequently milled and sieved in order to obtain particles of the order of 300-600 $\mu$m.

Characterization

The hydrogel synthesized in this configuration was characterized by analytical techniques reported in example 1.

In order to assess by FT-IR Laponite™ presence in materials 9L_tq and 9BL_tq, an analysis of the pure nanometric filler was performed beforehand. The FT-IR spectrum of pure Laponite™ consists of an intense absorption band at about 1000 cm$^{-1}$, due to the stretching of the Si-O group, and by a not very intense band at about 1637 cm$^{-1}$, attributable to the bending of the Si-O group; such absorptions are observed also in bentonite, as reported in Example 8. In samples 9L_tq and 9BL_tq an absorption increase at about 1008 cm$^{-1}$ is observed, already present in cellulose derivatives, as well as a small contribution at 1637 cm$^{-1}$, in particular in the Bentonite + Laponite mixed system (i.e., Example 9BL_tq).

Liquid absorption and retention data

The data of retention after centrifugation, related to the swelling in aqueous solution of 0,1 g of dry product, are reported in the table below. Having assessed beforehand that washings and drying by phase inversion in acetone afforded no improvements to material performance, said material was tested tel quel. Performance of CMCNa-based hydrogel with Laponite™ alone or with a Bentonite/Laponite™ mixture was assessed, as reported in the following Table:

| Product code | filler | Retention after centrifugation |
|---|---|---|
| 9L_tq | Laponite | 27 |
| 9BL_tq | Bentonite + Laponite | 32 |

The data of free absorption for 15 minutes and retention under load for 5 minutes (3 Kpa - 0.435 PSI) in saline solution (0.9% NaCl) by 0.5 g of product are reported in the following table:

| Product code | filler | Free swell | Retention under load |
|---|---|---|---|
| 9L_tq | Laponite | 48 | 47 |
| 9BL_tq | Bentonite + Laponite | 47 | 46 |

The data of absorption, under a load of 0.14 psi, of 0.3 g of dry product for 30 minutes in saline solution, are reported in the following table:

| Product code | filler | Absorption under load |
|---|---|---|
| 9L_tq | Laponite | 19 |
| 9BL_tq | Bentonite + Laponite | 22 |

Conclusions

This example highlights that the addition of inorganic fillers, in particular of nanometric sizes, such as Laponite™, affords substantial improvements to the performance of materials obtained from CMCNa, in particular with regard to the test of absorption under load. This parameter is of particular relevance, as it is precisely under load that usually the difference in terms of performance between an acrylic-based SAP and a polysaccharide-based one becomes clear: the latter, in fact, unlike acrylic SAPs, tends to form a soft jelly-like mass and be affected by the so-called gel-blocking effect when subjected to a load during its swelling. The approach undertaken, with a view to strengthening the absorbent gel under load with inorganic compounds apt to increase its gel-strength, proved particularly effective.

References

[1] Anbergen U, Opperman W, Polymer, 31, 1854 (1990).

[2] Denn W L, Ferguson G N, U.S. Pat. No. 3589364, (1971).

[3] Sannino A et al., International Patent Application WO2012170682.

**Claims**

1. A method for making superabsorbent polymers comprising the following steps:

   a) preparing an aqueous solution at pH equal or greater than 7.5 comprising alkylcellulose and at least one crosslinking agent, wherein said alkylcellulose has a concentration by weight between 2 and 10% and wherein said crosslinking agent is selected from boric acid, boronic acid, borax or an ester thereof in a concentration between 2 and 8% of said alkylcellulose;
   b) performing the crosslinking reaction obtaining a gel;
   c) drying the gel, wherein said alkylcellulose is selected from carboxymethylcellulose, hydroxyethylcellulose, methylhydroxyethylcellulose, methylhydroxypropylcellulose or mixtures thereof.

2. The method according to claim 1, wherein said crosslinking reaction in step a) is performed at a temperature between 25° and 80° C for a time of at least 1 hour.

3. The method according to claim 1 or 2, wherein in said aqueous solution $NaHCO_3$ is used as alkalinizing agent.

4. The method according to any of claims 1 to 3, comprising a preliminary step to step b) wherein said solution is subjected to a step of pre-evaporation.

5. The method according to claim 4, wherein said step of pre-evaporation is performed at a temperature of at least 45°C for a time ranging between 48 and 120 hours.

6. The method according to any of claims 1 to 5, wherein said aqueous solution comprises a molecular spacer and wherein said spacer is selected from a monosaccharide and/or a disaccharide and/or a polyol.

7. The method according to any of claims 1 to 6, wherein the pH is between 7.5 and 9.

8. The method according to any of claims 1 to 7, comprising a further step d) to reduce said polymer dried in step c) as powder.

9. The method according to any of claims 1 to 8, wherein said crosslinking agent is boric acid.

10. The method according to any of claims 1 to 9, wherein said aqueous solution prepared in step a) further comprises one or more organic and/or inorganic fillers selected from microcrystalline cellulose, talc, kaolin, silica, silicates, clays

11. The method according to claim 10 wherein said silicates are bentonites and/or laponites, wherein said silicates are added to said aqueous solution at a concentration between 0.1 and 2% by weight, preferably 0.5% by weight, of the total weight of the solution.

12. Superabsorbent polymers obtainable according to the method of any of claims 1 to 11.

13. Absorbent product for absorbing body fluids, **characterized by** the fact that its absorbent core comprises polymers according to claim 12, wherein said product is a diaper, a feminine towel, gauze or bandage.

14. A pharmaceutical composition comprising one or more active principles and polymers according to claim 12.

15. Absorbent product for food packaging comprising polymers according to claim 12.

**Patentansprüche**

1. Verfahren zum Herstellen von superabsorbierenden Polymeren, das die folgenden Schritte umfasst:

a) Herstellen einer wässrigen Lösung mit einem pH-Wert von ≥ 7,5, die Alkylcellulose und mindestens ein Vernetzungsmittel umfasst, wobei die Alkylcellulose bei einer Konzentration von 2 bis 10 Gewichtsprozent vorliegt und wobei das Vernetzungsmittel aus Borsäure, Boronsäure, Borax oder einem Ester davon ausgewählt wird und im Verhältnis zu der Alkylcellulose in einer Konzentration von 2 bis 8 % vorliegt;

b) Durchführen der Vernetzungsreaktion, wodurch ein Gel erhalten wird;

c) Trocknen des Gels, wobei die Alkylcellulose aus Carboxymethylcellulose, Hydroxyethylcellulose, Methylhydroxyethylcellulose, Methylhydroxypropylcellulose oder einer Mischung davon ausgewählt wird.

2. Verfahren gemäß Anspruch 1, wobei die Vernetzungsreaktion in Schritt a) bei einer Temperatur zwischen 25 und 80°C für mindestens eine Stunde durchgeführt wird.

3. Verfahren gemäß Anspruch 1 oder 2, wobei in der wässrigen Lösung $NaHCO_3$ als Alkalisierungsmittel verwendet wird.

4. Verfahren gemäß einem der Ansprüche 1 bis 3, das umfasst: einen Schritt vor dem Schritt b), in dem die Lösung einem Vorverdampfungsschritt ausgesetzt wird.

5. Verfahren gemäß Anspruch 4, wobei der Vorverdampfungsschritt bei einer Temperatur von mindestens 45°C für eine Dauer von 48 bis 120 Stunden durchgeführt wird.

6. Verfahren gemäß einem der Ansprüche 1 bis 5, wobei die wässrige Lösung einen Molekularabstandshalter umfasst und wobei der Abstandshalter aus einem Monosaccharid und/oder einem Disaccharid und/oder einem Polyalkohol ausgewählt wird.

7. Verfahren gemäß einem der Ansprüche 1 bis 6, wobei der pH-Wert zwischen 7,5 und 9 liegt.

8. Verfahren gemäß einem der Ansprüche 1 bis 7, das umfasst: einen weiteren Schritt d) zur Reduzierung des Polymers, das in Schritt c) zu Puder getrocknet wird.

9. Verfahren gemäß einem der Ansprüche 1 bis 8, wobei das Vernetzungsmittel Borsäure ist.

10. Verfahren gemäß einem der Ansprüche 1 bis 9, wobei die in Schritt a) hergestellte wässrige Lösung weiterhin umfasst: einen oder mehrere organische und/oder anorganische Filter, die aus mikrokristalliner Cellulose, Talkum, Kaolin, Kieselsäure, Silikaten, Tonen ausgewählt werden.

11. Verfahren gemäß Anspruch 10, wobei die Silikate Bentonite und/oder Laponite sind, wobei die Silikate der wässrigen Lösung mit einer Konzentration von 0,1 bis 2 Gewichtsprozent, vorzugsweise 0,5 Gewichtsprozent, bezüglich des Gesamtgewichts der Lösung hinzugefügt werden.

12. Superabsorbierende Polymere, die gemäß dem Verfahren gemäß einem der Ansprüche 1 bis 11 erhalten werden können.

13. Absorbierendes Produkt zum Absorbieren von Körperflüssigkeiten, **dadurch gekennzeichnet, dass** sein absorbierender Kern Polymere gemäß Anspruch 12 umfasst, wobei das Produkt eine Windel, Damenbinde, Gaze oder Bandage ist.

14. Pharmazeutische Zusammensetzung, die einen oder mehrere Wirkstoffe und Polymere gemäß Anspruch 12 umfasst.

15. Absorbierendes Produkt zum Verpacken von Lebensmitteln, das Polymere gemäß Anspruch 12 umfasst.

**Revendications**

1. Procédé de production de polymères superabsorbants, comprenant les étapes suivantes :

a) préparation d'une solution aqueuse à un pH égal ou supérieur à 7,5 comprenant une alkylcellulose et au moins un agent de réticulation, ladite alkylcellulose ayant une concentration en poids comprise entre 2 et 10% et ledit agent de réticulation étant choisi parmi l'acide borique, l'acide boronique, le borax et un ester de ceux-ci à une concentration comprise entre 2 et 8 % de ladite alkylcellulose ;

b) mise en oeuvre de la réaction de réticulation, ce qui donne un gel ;

c) séchage du gel,

dans lequel ladite alkylcellulose est choisie parmi la carboxyméthylcellulose, l'hydroxyéthylcellulose, la méthylhydroxyéthylcellulose, la méthylhydroxypropylcellulose et leurs mélanges.

2. Procédé selon la revendication 1, dans lequel ladite réaction de réticulation dans l'étape a) est mise en oeuvre à une température comprise entre 25 °C et 80 °C pendant une période d'au moins 1 heure.

3. Procédé selon la revendication 1 ou 2, dans lequel, dans ladite solution aqueuse, du $NaHCO_3$ est utilisé en tant qu'agent d'alcalinisation.

4. Procédé selon l'une quelconque des revendications 1 à 3, comprenant une étape préalable à l'étape b) dans laquelle ladite solution est soumise à une étape de pré-évaporation.

5. Procédé selon la revendication 4, dans lequel ladite étape de pré-évaporation est mise en oeuvre à une température d'au moins 45 °C pendant une période comprise entre 48 et 120 heures.

6. Procédé selon l'une quelconque des revendications 1 à 5, dans lequel ladite solution aqueuse comprend un écarteur moléculaire et dans lequel ledit écarteur est choisi parmi un monosaccharide et/ou un disaccharide et/ou un polyol.

7. Procédé selon l'une quelconque des revendications 1 à 6, dans lequel le pH est compris entre 7,5 et 9.

8. Procédé selon l'une quelconque des revendications 1 à 7, comprenant une autre étape d) pour réduire ledit polymère séché dans l'étape c) sous la forme d'une poudre.

9. Procédé selon l'une quelconque des revendications 1 à 8, dans lequel ledit agent de réticulation est l'acide borique.

10. Procédé selon l'une quelconque des revendications 1 à 9, dans lequel ladite solution aqueuse préparée dans l'étape a) comprend en outre une ou plusieurs charges organiques et/ou inorganiques choisies parmi la cellulose microcristalline, le talc, le kaolin, la silice, les silicates, les argiles.

11. Procédé selon la revendication 10, dans lequel lesdits silicates sont des bentonites et/ou des laponites, et dans lequel lesdits silicates sont ajoutés à ladite solution aqueuse à une concentration comprise entre 0,1 et 2 % en poids, de préférence de 0,5 % en poids, par rapport au poids total de la solution.

12. Polymères superabsorbants pouvant être obtenus conformément au procédé de l'une quelconque des revendications 1 à 11.

13. Produit absorbant pour absorber des fluides corporels, **caractérisé en ce que** son coeur absorbant comprend des polymères selon la revendication 12, lequel produit est une couche, une serviette hygiénique, une gaze ou un bandage.

14. Composition pharmaceutique comprenant un ou plusieurs principes actifs et des polymères selon la revendication 12.

15. Produit absorbant pour le conditionnement alimentaire, comprenant des polymères selon la revendication 12.

FIG.1

EP 2 956 178 B1

FIG.2

FIG.3

**REFERENCES CITED IN THE DESCRIPTION**

**Patent documents cited in the description**

- US 2008082068 A
- WO 2012170682 A

- US 3589364 A, Denn W L, Ferguson G N

**Non-patent literature cited in the description**

- **ANBERGEN U ; OPPERMAN W.** *Polymer,* 1990, vol. 31, 1854